# EUROPEAN PATENT APPLICATION

(11) **EP 4 681 628 A1**
(43) Date of publication of application: **21.01.2026**
(21) Application number: 25189226.1
(22) Date of filing: 14.07.2025
(51) Int. Cl.: A61B 5/06, A61B 5/287, A61B 5/00

(54) **MAGNETIC BASED SHEATH DETECTION**

(30) Priority: 15.07.2024 US 202418772581
(71) Applicant: Biosense Webster (Israel) Ltd., 2066717 Yokneam (IL)
(72) Inventor: BUCHNIK, Yael, 2066717 Yokneam (IL); GILNER, Vadim, 2066717 Yokneam (IL)
(74) Representative: Carpmaels & Ransford LLP

(57) **Abstract**

A method comprises receiving electrical signals from multiple coils embedded in a distal end assembly of a catheter delivered via a sheath inserted into a body of a patient, the electrical signals being received responsively to applying an external magnetic field to the distal end assembly. A change is determined in the value of an electrical signal outputted by a distal coil among the multiple coils. Based on the change in the value of the electrical signal, it is determined whether the distal end assembly is in a collapsed state inside the sheath or has begun emerging from the sheath and the distal coil is in at least partially expanded state outside the sheath. In response to detecting the change in the value, the value of the electrical signal from the distal coil is used for determining a position of a distal end of the sheath inside the body.

## Description

### FIELD OF THE DISCLOSURE

The present disclosure relates generally to invasive medical probes, and particularly to estimation of the location inside the human body of a distal end of a delivery sheath for an invasive medical probe.

### BACKGROUND OF THE DISCLOSURE

Techniques to determine a position and/or status of a probe were previously proposed in patent literature. For example, U.S. Patent Application Publication 2019/0343423 describes catheterization that is carried out by inserting a sheath into a human patient and moving a catheter having an electrode through the sheath lumen. A variation between a first threshold value and a second threshold value in electrical current through the electrode is identified. Responsively to the variation, it is reported that a portion of the catheter has transitioned between an in-sheath condition and an out-of-sheath condition. The sheath is defined and identified by the historical data of a readings of the magnetic sensor of the catheter during its movements.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a catheter-based electroanatomical (EA) mapping and ablation system, according to an example of the present disclosure;
Figs. 2A and 2B are pictorial illustrations that schematically show a three-coil layout and a six-coil layout for a flat catheter, according to some examples of the present disclosure;
Fig. 3 is a pictorial frontal view that schematically shows the catheter flat assembly of Fig. 1 in its rolled configuration inside the sheath, according to an example of the present disclosure;
Figs 4A, 4B, and 4C are three schematic depictions of the flat catheter assembly of Fig. 1: inside a distal end of a sheath, partially deployed out of the sheath, and fully deployed outside the sheath, respectively, according to an example of the present disclosure;
Fig. 5 is a flow chart that schematically illustrates a method and algorithm to detect the position of a distal end of a sheath using flat catheter assembly deployment, according to an example of the present disclosure;
Fig. 6 is an illustration of a perspective view of the flat catheter assembly of Fig. 1 (also called "end effector of the planar catheter"), according to an example of the present disclosure; and
Fig. 7 is an illustration of a catheter assembly comprising the end effector of Fig. 6, according to an example of the present disclosure.

### DETAILED DESCRIPTION OF EXAMPLES

### OVERVIEW

Diagnostic and therapeutic catheters are typically delivered through a sheath. An optimal deployment of the catheter may depend on the location of the distal edge of the sheath, i.e., from where the catheter exists. However, since sheaths are typically simple, flexible tubes without means of navigation, it may be difficult to determine the location of the distal end of a sheath in the body (e.g., inside the heart).

Examples of the present disclosure that are described hereinafter provide a technique that utilizes special layouts of magnetic position sensors (e.g., coils) on a flat catheter, the layouts possessing unique spatial symmetries of the set of coils to (i) determine the location of the distal end of a sheath inside the body during catheter assembly deployment outside the sheath, and (ii) determine the status of the catheter deployment (e.g., if it is still inside the sheath, partially out, or fully deployed outside the sheath). Using this real-time information may allow a physician to optimize catheter deployment (e.g., decide if the position of the sheath's distal end in the organ is optimal to proceed with full deployment of the catheter assembly).

The disclosed catheters have a flat distal-end assembly formed from certain self-expanding materials, such as flexible PCBs, which include three or more flat magnetic coils (e.g., wiring loops) embedded in the flexible PCB. The magnetic loops pick up the magnetic driving signals generated by a location tracking system's location pad (e.g., one located under the patient) and output respective electrical signals.

While the distal end assembly is inside the sheath and is rolled around its longitudinal axis, it yields pick-up signals from the magnetic sensors that are not suitable as position-indicative signals. However, the authors found that the nearly symmetrical coil layouts created by a rolled configuration causes these signals to fulfill certain relations that indicate that the flat assembly is either rolled in its entirety, or partially outside, the sheath.

Specifically, the authors noted that, as the flat distal-end assembly advances slightly beyond the distal end of the sheath, at least one distal coil outputs a changed signal compared to its output when rolled inside the sheath. A processor of the tracking system uses the changed signals to determine, to a certain accuracy (e.g., a few millimeters), the position of the distal end of the sheath inside the body. To this end, the changed signal should have a value similar to (e.g., nearly as large as) the standard counterpart position signal, although it will usually differ from that of a fully deployed coil.

When the assembly is fully deployed (e.g., in its self-expanded shape outside the sheath) all of the coils output standard position-inductive signals. The position-tracking system uses these signals to accurately determine the location of the distal end assembly inside the body.

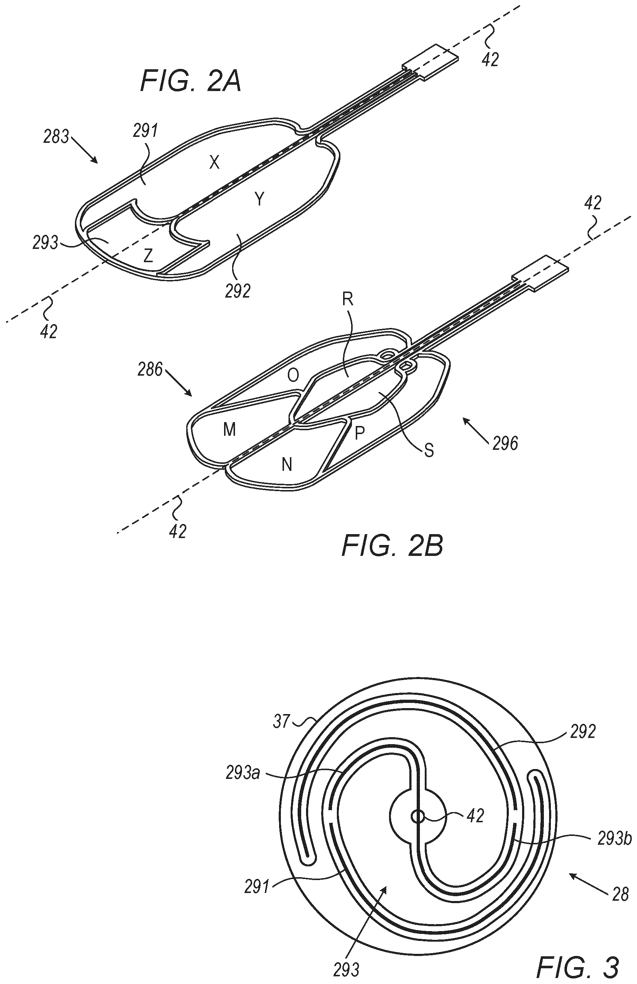

In one example, the tracking system's processor receives signals from three magnetic sensors (e.g., X, Y designated side loops, and a Z called distal loop, as illustrated in Fig. 2A) disposed over a flat distal end assembly of the catheter while it is being delivered through a sheath. The system monitors real-time changes in the output of each of the three magnetic loops on the assembly.

While the distal end assembly is in its rolled configuration inside the sheath, portions of any two side loops (X, Y) face one another about any external magnetic field direction. As a result, the output signals (SX, SY) of side loops (X, Y) are of equal magnitude but opposite in sign: SX = -SY. Signal output SZ of distal loop Z is largely zero due to its essential asymmetric spatial layout about the magnetic field direction, where each contribution by one of its portions is canceled by a respective portion facing it, relative to the magnetic field.

More generally, the corresponding relationship of signals (SX, SY and SZ) inside the sheath can be written as SZ=ε, SX=ε+A, and SY=ε-A, with ε being small or zero. As long as this relationship is maintained, the processor can determine that the flat distal end assembly is rolled and therefore must be in the sheath.

As the flat distal end assembly slightly exits the distal end of the sheath, distal loop Z begins to self-expand into its unrolled flat shape, and the output SZ of distal loop Z becomes larger than a predefined threshold (e.g., larger than 25ε). At this point, the location system can use the position indication obtained from the distal loop to estimate the position of the distal end of the sheath inside the body (e.g., inside the heart).

When the flat assembly is fully deployed, all loops are in a self-expanded flat shape. In this state, side loop signals (SX, SY) are no longer opposite in sign, and, at this point, the position indication of the assembly obtained using the three loops is highly accurate.

### SYSTEM DESCRIPTION

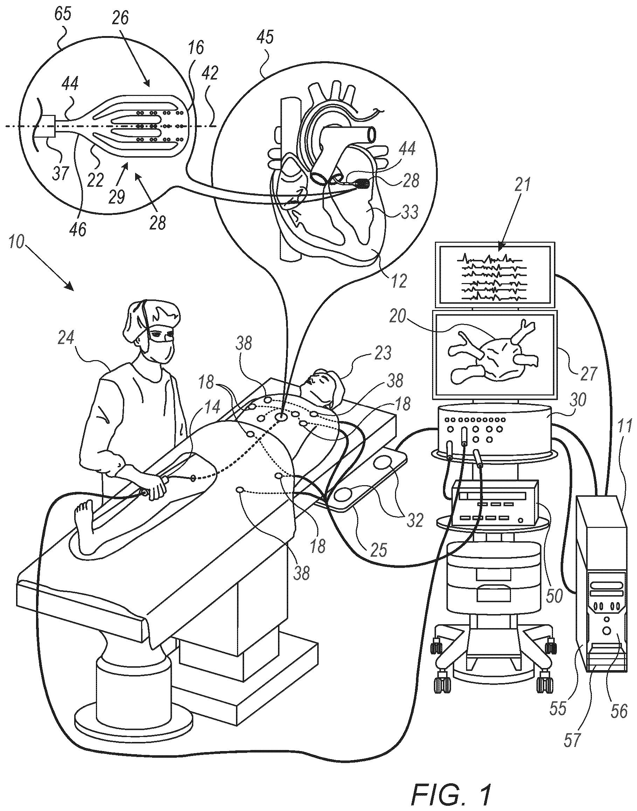

Fig. 1 is a schematic, pictorial illustration of a catheter-based electroanatomical (EA) mapping and ablation system 10, according to an example of the present disclosure. System 10 includes a delivery sheath 37 percutaneously inserted, by physician 24, through the patient's vascular system into a chamber or vascular structure of a heart 12. Thereafter, a flat catheter 14 (illustrated in inset 45) is inserted into delivery sheath 37 to be deployed outside of sheath 37 (e.g. in a blood pool of a ventricle 33) and then advanced to a desired tissue location.

In Fig. 1, a physician 24 advances the flat type of expandable distal-end assembly 28 (also called hereinafter "expandable distal-end assembly 28") fitted on a shaft 44 of catheter 14 into contact with the heart wall for EA sensing a target site in heart 12.

As seen in inset 65, flat assembly 28 includes multiple functional electrodes 26 optionally distributed over a plurality of splines 22 at expandable distal-end assembly 28 and configured to sense IEGM signals. Assembly 28 has a longitudinal axis 42, which is parallel to a distal end 46 of shaft 44 and is an axis of symmetry of assembly 28.

Assembly 28 includes a magnetic position sensor 29 comprising three coils seen in Fig. 2A. The coils are embedded in splines 22 and are used for tracking the position and/or orientation of expandable distal end assembly 28. One of the coils is located closer to a distal edge 16 of assembly 28 and is therefore named "the distal coil." Magnetic position sensor 29 is operated together with an external location pad 25 that includes a plurality of magnetic coils 32 configured to generate magnetic fields in a predefined working volume. Using the operation with external location pad 25 (each coil using a different frequency), the processor can determine the position of sensor 29 on a coordinate system of the position tracking system.

Details of the magnetic-based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish a location reference for location pad 25 as well as impedance-based tracking of functional electrodes 26. For impedance-based tracking, electrical current is directed toward electrode 26 and sensed at electrode skin patches 38, such that the location of each electrode can be triangulated via electrode patches 38.

Details of the impedance-based location tracking technology are described in US Patent Nos. 7,536,218; 7,756,576; 7,848,787; 7,869,865; and 8,456,182.

A recorder 11 displays electrograms 21 captured with body surface ECG electrodes 18 and intracardiac electrograms (IEGM) captured with functional electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to a subset of the plurality of electrodes 26 at the distal assembly 28 of catheter 14 configured for ablating. Energy produced by ablation energy generator 50 may include, but is not limited to, radiofrequency (RF) energy or pulsed-field ablation (PFA) energy, including monopolar or bipolar high-voltage DC pulses that may be used to effect irreversible electroporation (IRE), or combinations thereof.

Patient interface unit (PIU) 30 is configured to establish electrical communication between catheters, electrophysiological equipment, power supply and a workstation 55 for controlling the operation of system 10. Electrophysiological equipment of system 10 may include, for example, multiple catheters, location pad 25, body surface ECG electrodes 18, electrode patches 38, ablation energy generator 50, and recorder 11. Optionally, and preferably, PIU 30 additionally includes processing capability for implementing real-time computations of catheter locations and for performing ECG calculations.

Workstation 55 includes memory 57, processor unit 56 with memory or storage with appropriate operating software loaded therein, and user interface capability. Workstation 55 may provide multiple functions, optionally including (i) modeling endocardial anatomy in three-dimensions (3D) and rendering the model or anatomical map 20 for display on a display device 27, (ii) displaying on display device 27 activation sequences (or other data) compiled from recorded electrograms 21 in representative visual indicia or imagery superimposed on the rendered anatomical map 20, (iii) displaying real-time location and orientation of multiple catheters within the heart chamber, and (iv) displaying on display device 27 sites of interest such as places where ablation energy has been applied. One commercial product embodying elements of system 10 is available as the CARTO^{™} 3 System, available from Biosense Webster, Inc., 31A Technology Drive, Irvine, CA 92618.

### FLAT CATHETER COILS LAYOUTS

Figs. 2A and 2B are pictorial illustrations that schematically show a three-coil layout 283 and a six coil layout 286 for a flat catheter, according to some examples of the present disclosure.

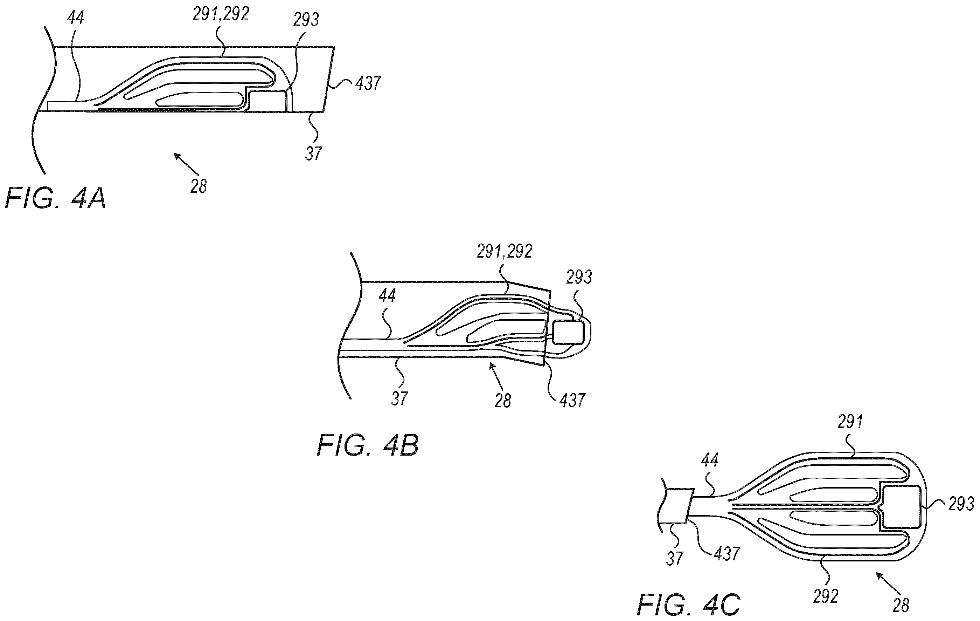

Fig. 2A shows the layout 283 of three (X, Y, Z) coils (291, 292, 293) of sensor 29 of distal end assembly 28 that is further described in Figs. 3 and 4. As seen, the layout comprises a first and second side coils (291, 292) located on respective first and second sides of the distal end assembly, and a distal coil 293.

When the distal end assembly is rolled inside the sheath, loops (291, 292, 293) are rolled over longitudinal axis 42 and form a largely asymmetrical spatial layout about the magnetic field direction.

As the flat distal end assembly slightly exits the distal the of the sheath, only distal loop Z begins to self-expand into its unrolled flat shape.

When the flat assembly is fully deployed, the side loops also self-expand into a flat shape.

Fig. 2B shows layout 286 of six (M, N, O, P, R, S) coils 296. A larger number of coils may allow the position tracking system to determine the status of the catheter deployment off the sheath in greater detail.

Fig. 3 is a pictorial frontal view that schematically shows catheter 14 flat assembly 28 of Fig. 1 in its rolled configuration inside sheath 37, according to an example of the present disclosure. As seen, flat assembly 28 is rolled about longitudinal axis 42 with side loops 291 and 292 showing opposite facets to any magnetic field. This results in their pick-up signals SX and SY to largely fulfilling the relation SX = -SY.

The two portions 293a and 293b of distal coil 293 also largely show opposite facets to any magnetic field. This results in coil 293 pick-up signal SZ to largely fulfill the relation SZ=ε (e.g., ε~0).

### MAGNETIC BASED SHEATH DETECTION USING FLAT CATHETER DEPLOYMENT

Figs 4A, 4B, and 4C are schematic depictions of the flat catheter assembly 28 of Fig. 1 inside a distal end of sheath 37, partially deployed out of sheath 37, and fully deployed outside sheath 37, respectively, according to an example of the present disclosure.

Table 1 below lists the signal values, or relations, that coils (291, 292 and 293) output/hold in any of the three deployment configurations of Figs 4A-4C.

**Table 1**

| **Figure** | **Assembly Deployment Stage** | **Upper Loop** | **Side Loops** |
|---|---|---|---|
| **4A** | Inside sheath | SZ = ε | SX = -SY |
| **4B** | Partially outside sheath | SZ ≠ ε | SX = -SY |
| **4C** | Outside sheath | SZ ≠ ε | SX ≠ -SY |

Fig. 4A shows, schematically, that while being rolled inside sheath 37, side coils 291 and 292 face each other, thereby generating pick-up signals largely equal in magnitude but opposite in sign. At the same time, the two sides of distal coil 293 also face each other, thereby generating a largely zero pick-up signal.

Fig. 4B shows, schematically, that while assembly 28 is partially rolled inside sheath 37 (i.e., partially outside sheath 37), side coils 291 and 292 still face each other, thereby generating pick-up signals largely equal in magnitude but opposite in sign. At the same time, distal coil 293 is already partially expanded off sheath edge 437 into its flat shape, and therefore coil 293 generates a largely true position signal SZ.

Fig. 4C shows, schematically, assembly 28 fully outside sheath 37 at its self-expanded flat shape. In this case side coils 291 and 292 are expanded off sheath edge 437 and generate different true position signals (e.g., different magnitude position signals but with the same sign). Distal coil 293 is in its self-expanded flat shape and generates a true position signal SZ.

### METHOD OF MAGNETIC BASED SHEATH DETECTION USING FLAT CATHETER DEPLOYMENT

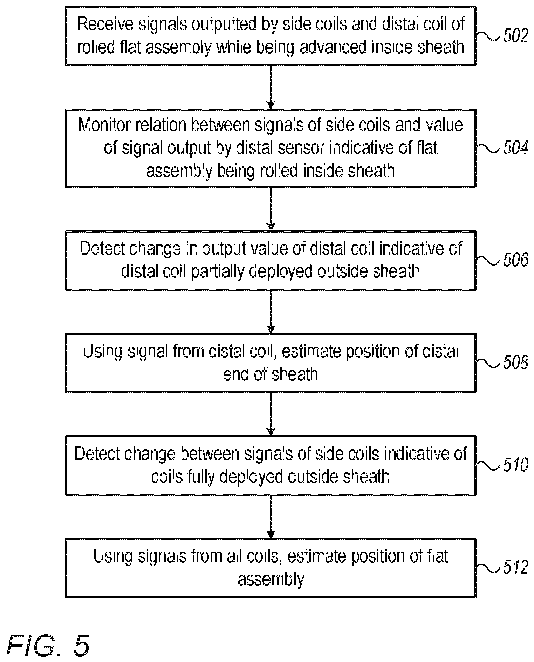

Fig. 5 is a flow chart that schematically illustrates a method and algorithm to detect the position of a distal end of sheath 37 using flat catheter assembly 28 deployment, according to an example of the present disclosure. The algorithm, according to the present example, carries out a process that begins at a signal receiving step 502, with processor 56 receiving electrical signals outputted by side coils (291, 292) and distal coil 293 of assembly 28 rolled inside sheath 37 as the assembly is being advanced inside sheath 37.

At signals relation monitoring step 504, processor 56 monitors in real-time the relation between the electrical signals received in step 502 from side coils 291 and 292, and the value of the signal from the distal coil 293, such as monitoring that these signals fulfill the entries given in Table 1 for Fig. 4A when the catheter is in its rolled configuration inside the sheath.

At signal value change detection step 506, the processor detects a change in the electrical signal value outputted by distal coil 293, indicative of a change in shape of flat assembly 28 as it starts exiting sheath 37. The amount of a detected change indicative of distal coil 293 being partially outside the sheath and having at least a partially self-expanded shape with the corresponding new entry is given in Table 1 for Fig. 4B.

At sheath position estimation step 508, the processor uses the first substantial (e.g., larger than a predefined threshold) position signal from the at least a partially self-expanded distal coil 293 to estimate a position of the distal end of sheath 37 inside the body.

Next, at signals relation change detection step 510, the processor detects a change in the relation between the signals from coils (291, 292), indicative of a further change in shape of the flat assembly. For flat catheter assembly 28, this amounts to detecting a change indicative that all distal coils (291, 292 and 293) are deployed in full outside the sheath, with the corresponding new relations, is given in Table 1 for Fig. 4C.

At assembly position estimation step 512, the processor uses the position signals from all three coils to estimate a position of flat assembly 28 inside the body. This initial estimation can also be used to corroborate sheath 37 location estimation of step 508.

The example flow chart shown in Fig. 5 is simplified for the sake of conceptual clarity. For example, additional intermediate steps (e.g., changes in relations) can be considered, depending on coil layout and on workflow. Additional steps may also include retracting the catheter back into sheath 37 based on the sheath's distal end location (determined in step 508) as being deemed unusable, whereupon the sheath is moved to a more optimal location before fully deploying assembly 28.

### DOUBLE-SIDED ENCAPSULATED PLANAR CATHETER

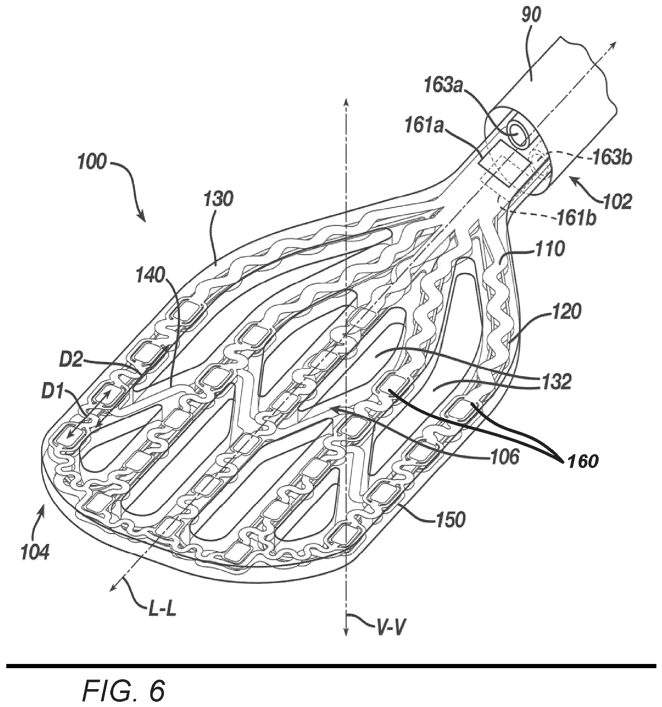

Fig. 6 is an illustration of a perspective view of the flat catheter assembly 28 of Fig. 1 (also called "end effector 100 of the planar catheter"), according to an example of the present disclosure. Double-sided end effector 100 is coupled to a catheter shaft 90. Importantly, the end effector 100 has an overall flat profile and is disposed of with electrodes 160 on its two opposing (along axis V-V) planar facets, e.g., on tines 106. The two opposing facets' layouts may be identical or different.

There can be provided a plurality of pairs of electrodes 160 disposed on the flexible circuits on the two opposing facets of the effector 100, the electrodes being spaced apart a first predetermined longitudinal distance D1 and each pair of electrodes 160 spaced apart from adjacent pairs of electrodes 160 a second predetermined longitudinal distance D2, the second predetermined longitudinal distance D2 being greater than the first predetermined longitudinal distance D1.

Electrodes 160 can sense tissue signals or transmit energy (AC or DC) from an energy generator to the tissues. At least a portion of electrodes 160 can be axially aligned, orthogonal to the longitudinal axis L-L, with at least a position of the electrodes defining pairs of opposite-facing electrodes.

End effector 100 can include a framework 120, non-conductive flexible layers 130, and flexible circuits 110 and 150. The contiguous mass of flexible, non-conductive material 130 can have portions 132 removed. Portions 132 can also be formed in one or all of the flexible circuits and the framework 120. Portions 132 can pass through all layers of the multi-layered end effector 100, or just some of the layers. Portion 132 can be included to increase the ability of the end effector 100 to fold or bend into a reduced delivery configuration to allow the end effector 100 to be passed through a delivery catheter.

The flexible circuits 110 and 150 can extend along a longitudinal axis L-L from a proximal portion 102 to a distal portion 104 of the end effector 100 and can each include two opposing faces. Framework 120 can include two opposite sides and can extend along the longitudinal axis L-L generally parallel to flexible circuits 110 and 150. Non-conductive flexible layers 130 can at least partially encapsulate the first flexible circuits 110 and 150 and the framework 120.

In some examples, flexible circuit layers 110 and 150 can be made primarily of polyimide. In other examples, it can be made of any of biocompatible polyimides, glass-reinforced epoxy laminate materials, copper, or graphene, alone or in combination. The flexible circuit layers can include conductive traces.

The end effector 100 includes a location sensing coil layer 140 containing a plurality of coils, such as coils (291, 292 293) of Fig. 2A, lying generally parallel to the framework 120 and separated from framework 120 by another of non-conductive flexible layers 130. The coils can, in some examples, be coplanar.

Far-field signals (including noise or artifacts) can be reduced or canceled out for the overall end effector with reference electrodes 161a and 161b disposed on the two opposing sides near the proximal base 102 of the framework 100 such that the reference electrodes are not in contact with tissues and only with blood. The reference electrodes 161 are preferably exposed to the ambient blood environment but can be encased in the polymer so that the reference electrodes are not exposed through the non-conductive layer.

Irrigation can be provided with two irrigation ports 163a and 163b (one at each side) in fluid communication with an irrigation line (not shown) disposed in catheter shaft 90. Instead of an irrigation line separate from the catheter shaft 90, a lumen can be formed via extrusion of the catheter shaft 90 to provide for a lumen channel. It is noted that ports 163 can be configured to have a sufficient flow diverter characteristic for irrigation fluid to cover the electrodes during irrigation flow so as to prevent or reduce thrombus formations.

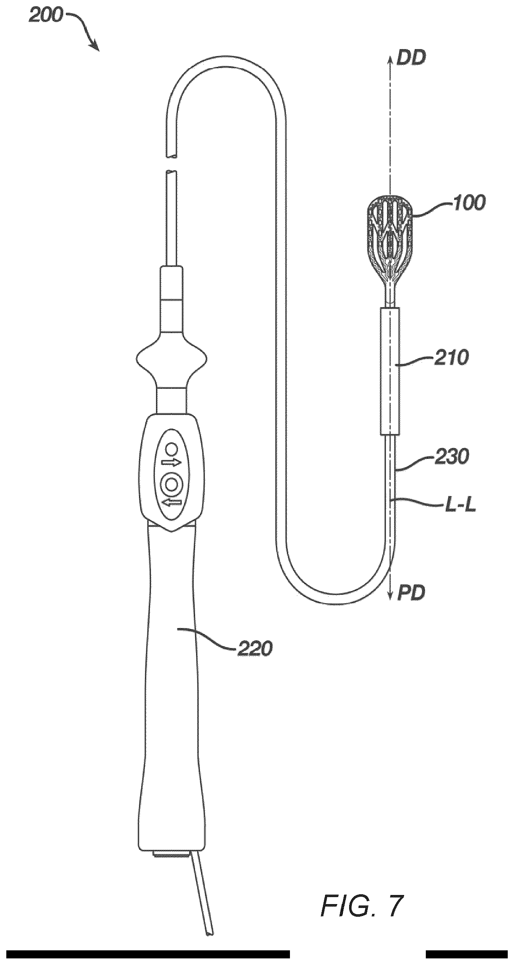

Details of the spacing of the electrodes can be found in U.S. Provisional Patent Application S.N.63/406,673 (Attorney Docket No. BIO6749USPSP3) filed on September 14, 2022, and incorporated by reference in its entirety into this application as if set forth in full and attached in the Appendix to priority application U.S. Provisional Patent Application No. 63/505,764 (Attorney Docket No. 253757.000380 BIO6846USPSP1) filed June 02, 2023.The present disclosure provides a catheter assembly 200 as shown in Fig. 7, which can include a tubular member 230 (also called "shaft" 230) extending along a longitudinal axis L-L and configured to deliver end effector 100 to an out of a sheath 210. A physician 24 can manipulate the catheter 200 with a handle 220. Appropriate examples for catheter assembly 200 and its subcomponents such as handle 220, sheath 210, tubular member 230, and others not mentioned herein are described in US Patent publication No. 2021/0369339, which is incorporated by reference in its entirety into this application as if set forth in full and attached in the Appendix to priority application U.S. Provisional Patent Application No. 63/505,764 (Attorney Docket No. 253757.000380 BIO6846USPSP1) filed June 02, 2023.

### EXAMPLES

### Example 1

A method comprises receiving electrical signals from multiple coils (291, 292, 293) embedded in a distal end assembly (28, 283) of a catheter (14) delivered via a sheath (37) inserted into a body of a patient, the electrical signals being received responsively to applying an external magnetic field to the distal end assembly (28, 283). A change is determined in the value of an electrical signal outputted by a distal coil (293) among the multiple coils. Based on the change in the value of the electrical signal, it is determined whether the distal end assembly (28, 283) is in a collapsed state inside the sheath (37) or has begun emerging from the sheath and the distal coil (293) is in at least partially expanded state outside the sheath (37). In response to detecting the change in the value, the value of the electrical signal from the distal coil (293) is used for determining a position of a distal end of the sheath (37) inside the body.

### Example 2

The method according to example 1, and comprising, determining a relation between the electrical signals outputted by two or more side coils (291, 292) among the multiple coils (291, 292, 293), and, upon detecting a change in the relation, based on the electrical signals, identifying that the distal end assembly (28, 283) has fully emerged from the sheath (37) and is fully expanded.

### Example 3

The method according to example 2, and comprising, upon detecting a change in the relation, calculating, based on the electrical signals, a position of the expanded distal end assembly (28, 283) within the body.

### Example 4

The method according to example 2, wherein the distal end assembly (28, 283) comprises first and second side coils (291, 292) located on respective first and second sides of the distal end assembly, and wherein determining the relation comprises determining a relation between the electrical signals output by the first and second side-coils (291, 292).

### Example 5

The method according to example 1, and comprising determining the change in the value of the electrical signal output by the distal coil (293) has occurred if the change exceeds a predefined threshold value.

### Example 6

The method according to example 1, wherein the distal coil (293) is located at a distal edge of the distal end assembly (28, 283).

### Example 7

A system includes an interface (30) and a processor (56). The interface (30) is configured for receiving electrical signals from multiple coils (291, 292, 293) embedded in a distal end assembly (28, 283) of a catheter (14) delivered via a sheath (37) inserted into a body of a patient, the electrical signals being received responsively to applying an external magnetic field to the distal end assembly (28, 283). The processor (56) is configured to (i) determine a change in value of an electrical signal outputted by a distal coil (293) among the multiple coils, (ii) based on the change in the value of the electrical signal, determine whether the distal end assembly (28, 283)is in a collapsed state inside the sheath (37) or has begun emerging from the sheath and the distal coil (293) is in at least partially expanded state outside the sheath, and (iii) in response to detecting the change in the value, use the value of the electrical signal from the distal coil (293) to determine a position of a distal end of the sheath (37) inside the body.

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A method, comprising:
receiving electrical signals from multiple coils (291, 292, 293) embedded in a distal end assembly (28, 283) of a catheter (14) delivered via a sheath (37) inserted into a body of a patient, the electrical signals being received responsively to applying an external magnetic field to the distal end assembly (28, 283);
determining a change in value of an electrical signal outputted by a distal coil (293) among the multiple coils;
based on the change in the value of the electrical signal, determining whether the distal end assembly (28, 283) is in a collapsed state inside the sheath (37) or has begun emerging from the sheath and the distal coil (293) is in at least partially expanded state outside the sheath (37); and
in response to detecting the change in the value, using the value of the electrical signal from the distal coil (293) to determine a position of a distal end of the sheath (37) inside the body.

2. The method according to claim 1, and comprising, determining a relation between the electrical signals outputted by two or more side coils among the multiple coils (291, 292, 293), and, upon detecting a change in the relation, based on the electrical signals, identifying that the distal end assembly (28, 283) has fully emerged from the sheath (37) and is fully expanded.

3. The method according to any one of claims 1-2, and comprising, upon detecting a change in the relation calculating, based on the electrical signals, a position of the expanded distal end assembly (28, 283) within the body.

4. The method according to any one of claims 1-3, wherein the distal end assembly (28, 283) comprises first and second side coils (291, 292) located on respective first and second sides of the distal end assembly (28, 283), and wherein determining the relation comprises determining a relation between the electrical signals output by the first and second side-coils (291, 292).

5. The method according to any one of claims 1-4, and comprising determining the change in the value of the electrical signal output by the distal coil (293) has occurred if the change exceeds a predefined threshold value.

6. The method according to any one of claims 1-5, wherein the distal coil (293) is located at a distal edge of the distal end assembly (28, 283).

7. A system, comprising:
an interface (30) configured for receiving electrical signals from multiple coils (291, 292, 293) embedded in a distal end assembly (28, 283) of a catheter (14) delivered via a sheath (37) inserted into a body of a patient, the electrical signals being received responsively to applying an external magnetic field to the distal end assembly (28, 283); and
a processor (56), which is configured to:
determine a change in value of an electrical signal outputted by a distal coil (293) among the multiple coils;
based on the change in the value of the electrical signal, determine whether the distal end assembly (28, 283) is in a collapsed state inside the sheath (37) or has begun emerging from the sheath (37) and the distal coil (293) is in at least partially expanded state outside the sheath (37); and
in response to detecting the change in the value, use the value of the electrical signal from the distal coil (293) to determine a position of a distal end of the sheath (37) inside the body.

8. The system according to claim 7, wherein the processor (56) is further configured to determine a relation between the electrical signals outputted by two or more side coils (291, 292) among the multiple coils, and, upon detecting a change in the relation, based on the electrical signals, identify that the distal end assembly (28, 283) has fully emerged from the sheath (37) and is fully expanded.

9. The system according to any one of claims 7-8, wherein the processor (56) is further configured to, upon detecting a change in the relation calculate, based on the electrical signals, a position of the expanded distal end assembly (28, 283) within the body.

10. The system according to any one of claims 7-9, wherein the distal end assembly (28, 283) comprises first and second side coils (291, 292) located on respective first and second sides of the distal end assembly (28, 283), and wherein the processor (56) is configured to determine the relation by determining a relation between the electrical signals output by the first and second side-coils (291, 292).

11. The system according to any one of claims 7-10, wherein the processor (56) is further configured to determine the change in the value of the electrical signal output by the distal coil (293) has occurred if the change exceeds a predefined threshold value.

12. The system according to any one of claims 7-11, wherein the distal coil (293) is located at a distal edge of the distal end assembly (28, 283).
